# FASCICULE DE BREVET EUROPEEN

(11) **EP 3 908 169 B1**
(45) Date de publication et mention de la délivrance du brevet: **23.09.2026**
(21) Numéro de dépôt: 20710507.3
(22) Date de dépôt: 11.03.2020
(51) Int. Cl.: A61B 1/00, A61B 1/267

(54) **BOUGIE D'INTUBATION**
INTUBATIONSBOUGIE
INTUBATION BOUGIE

(30) Priorité: 11.03.2019 FR 1902466
(43) Date de publication de la demande: 17.11.2021
(73) Titulaire: Prodol Meditec S.A., 48930 Las Arenas, Vizcaya (ES)
(72) Inventeur: LUCCHINA, Pascal, 33200 BORDEAUX CAUDERAN (FR); CHECCARONI, Stéphane, 06000 NICE (FR); DHONNEUR, Gilles, 94420 LE PLESSIS TREVISE (FR)
(74) Mandataire: Carlos Hernando, Borja
(86) Numéro de dépôt international: PCT/EP2020/056433
(87) Numéro de publication internationale: WO 2020/182851

(56) Documents cités:
- EP-A1- 2 875 841
- US-A1- 2014 018 626
- US-A1- 2019 014 980

## Description

### DOMAINE TECHNIQUE

La présente invention est relative à un dispositif d'intubation trachéale. Elle trouve au moins une application particulièrement avantageuse pour des intubations difficiles de patients présentant une glotte désaxée. Elle permet en outre de réaliser des intubations rapides et atraumatiques.

### ARRIERE-PLAN TECHNOLOGIQUE

Dans le domaine médical, pour poser une sonde d'intubation dans les voies aériennes supérieures (VAS) d'un patient, un guide d'intubation également dénommé mandrin d'intubation ou encore bougie d'intubation est généralement utilisé.

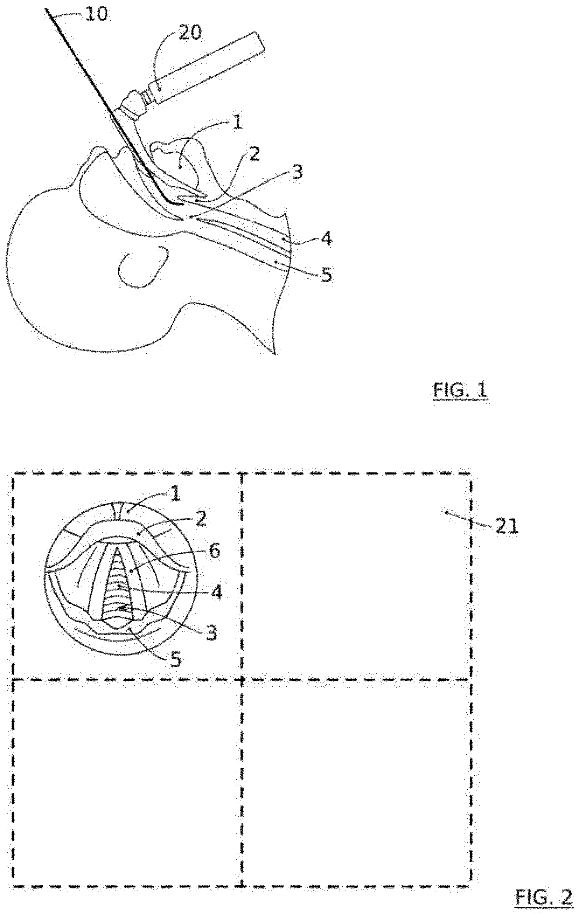

Tel qu'illustré à la figure 1, la bougie d'intubation 10 est introduite dans le larynx avec l'assistance d'un laryngoscope 20, puis dans la trachée 4, pour guider la sonde d'intubation dans les VAS.

Une telle bougie d'intubation 10 se présente sous forme d'une tige flexible et/ou malléable pouvant présenter une longueur d'environ 50 cm à 70 cm pour atteindre la trachée 4 du patient en respectant l'anatomie courbe des VAS.

La bougie d'intubation 10 peut également présenter une partie courbe et béquillée au niveau de son extrémité distale afin de faciliter la pénétration de la bougie et, in fine, de la sonde dans l'orifice d'entrée de la trachée 4, appelé glotte 3.

Par exemple, la bougie d'Eschmann est équipée d'une extrémité béquillée sur 1,5 à 2 cm réalisant un angle d'environ 30° avec l'axe longitudinal principal de la bougie.

Selon un autre exemple, le document WO 2016/044438 A1 divulgue un dispositif possédant plusieurs sections flexibles ou malléables permettant de le courber dans différentes configurations de bougie d'intubation.

Ce dispositif possède en outre une section distale pourvue d'une extrémité courbée et arrondie.

Le praticien chargé d'introduire la bougie cherche un canal de passage dans les VAS du patient.

Lors de la progression de la bougie dans les VAS, il cherche en particulier à sentir avec l'extrémité distale de la bougie des contacts anatomiques et/ou des blocages contre les structures cartilagineuses du larynx, de la glotte, de la trachée puis des bronches, qui sont situées à une distance d'environ 35 à 40 cm de la bouche.

Une telle progression est généralement traumatique pour le patient.

Afin de faciliter l'intubation trachéale, des vidéolaryngoscopes (VL) équipés de caméras permettent de visualiser la progression de la bougie et/ou de la sonde d'intubation au travers du canal de passage dans les VAS.

Une telle visualisation offre un champ de vision endoscopique généralement désaxé par rapport au canal de passage de la sonde d'intubation.

Par ailleurs, comme illustré à la figure 2, cette visualisation peut conduire, chez certains patients, à observer une glotte 3 particulièrement désaxée vis-à-vis du champ de vision 21 endoscopique du vidéolaryngoscope. La glotte 3 reste cependant visible, généralement dans une position haute plus ou moins latéralisée relativement au champ de vision 21 endoscopique du VL.

Dans ce cas, il est nécessaire de guider la sonde d'intubation souple pour éviter que celle-ci ne soit déviée en dessous de la glotte vers l'œsophage 5.

Une telle intubation correspond à une situation d'intubation difficile nécessitant une bougie d'intubation maniable, agile, facilement pilotable et atraumatique, permettant une intervention rapide.

Les bougies d'intubation citées plus haut ne sont pas adaptées à de telles situations d'intubation difficiles.

En particulier, la maniabilité de telles bougies d'intubation est souvent frustre, au mieux correcte. Leur pilotabilité est très limitée. Leur agilité est quasi-nulle.

Ces bougies sont en outre généralement blessantes.

Des lésions de la muqueuse antérieure de la trachée et des accidents hémorragiques et infectieux secondaires à des plaies trachéales pénétrantes (perforations) ont été observés avec de telles bougies d'intubation.

Ces bougies ne permettent donc pas une intubation rapide, la peur de blesser le patient étant constante pour le praticien qui hésite à réaliser certains gestes pour ouvrir le canal de passage vers la trachée. Le patient est en détresse respiratoire tout au long de l'intubation, jusqu'à ce que la sonde d'intubation soit effectivement en place dans les bronches.

Les difficultés rencontrées avec ces bougies peuvent donc créer des situations d'une extrême gravité avec des risques de lésions et de complications ultérieures pour le patient.

Par ailleurs, leur prix est élevé et constitue une part très majoritaire du coût total de l'intubation trachéale.

US2014/018626A1 divulgue un dispositif d'intubation extensible comprenant un assemblage de tubes creux télescopiques, avec un passage fluidique continu et des tubes pouvant se déplacer les uns par rapport aux autres. L'extrémité distale peut être guidée par des fils de commande afin de modifier la longueur et/ou la courbure du dispositif pendant l'intubation.

Un objet de la présente invention est de pallier au moins en partie certains des inconvénients mentionnés ci-dessus.

Plus particulièrement, selon un aspect, l'invention vise à proposer un dispositif d'intubation permettant un accès trachéal rapide et atraumatique, adapté aux situations d'intubation difficiles.

Selon un autre aspect, l'invention vise à proposer un procédé de fabrication d'un tel dispositif d'intubation, permettant de limiter le coût de fabrication de ce dispositif.

### RESUME

Un premier aspect de l'invention concerne une bougie d'intubation sous forme de tige comprenant un corps présentant un premier axe longitudinal, dit axe terminal, et une partie distale s'étendant depuis le corps.

Selon l'invention, la partie distale comprend au moins trois segments distincts entre eux, disposés successivement et configurés de sorte à conférer à la partie distale une souplesse croissante depuis une première extrémité de la partie distale solidaire du corps jusqu'à une deuxième extrémité libre de la partie distale, opposée à la première extrémité et destinée à pénétrer les voies aériennes supérieures d'un patient.

Selon l'invention les au moins trois segments sont articulés les uns par rapport aux autres, par au moins une articulation conformée entre deux segments adjacents parmi les au moins trois segments, et les au moins trois segments forment un noyau surmoulé par une gaine lequel est formée en un matériau différent de celui desdits au moins trois segments.

La bougie d'intubation selon l'invention permet ainsi de combiner une bonne pilotabilité grâce au corps de la bougie et une agilité importante au niveau de la partie distale grâce aux segments conférant une souplesse croissante à la partie distale jusqu'à sa deuxième extrémité libre.

La bougie d'intubation selon l'invention présente donc une grande maniabilité permettant de franchir différentes structures anatomiques en particulier lors d'une intubation difficile.

La souplesse croissante de la partie distale de la bougie favorise en outre une utilisation atraumatique.

Selon un mode de réalisation préféré de l'invention, la partie distale est configurée pour atteindre des glottes difficiles d'accès, en particulier des glottes décentrées dans un champ de vision endoscopique d'un vidéolaryngoscope.

Les segments, de préférence en élastomère, de la partie distale sont désaxés, les uns par rapport aux autres de façon à former une corne. Cette corne permet avantageusement d'atteindre des glottes décentrées par rapport à l'axe terminal du corps de la bougie.

Cette corne permet en outre de rattraper le désaxage entre l'axe optique de la caméra du vidéo laryngoscope et le canal de passage de la bougie.

Selon une possibilité particulièrement avantageuse et optionnelle, le corps de la bougie comprend en outre des cannelures longitudinales. Ces cannelures longitudinales permettent de limiter les frottements entre la sonde d'intubation et le corps de la bougie. La rotation de la bougie dans la sonde d'intubation est ainsi facilitée. La pilotabilité de la bougie est améliorée.

Ces cannelures peuvent également coopérer avec une gaine de ventilation optionnelle ajustée sur le pourtour du corps de la bougie. Elles forment dans ce cas des canaux pour le passage d'un fluide, par exemple de l'oxygène.

Cela permet avantageusement d'injecter de l'oxygène ou de ventiler le patient en cours d'intubation, avant la mise en place finale de la sonde d'intubation dans les bronches.

La présente demande décrit aussi un procédé de fabrication d'une bougie d'intubation selon le premier aspect de l'invention comprenant au moins la ou les étapes suivantes :
Mouler, de préférence par injection, le corps et les au moins trois segments de la partie distale de la bougie simultanément en un premier matériau,

De préférence, surmouler les au moins trois segments de la partie distale de sorte à former une partie distale comprenant un noyau en le premier matériau et une gaine en un deuxième matériau différent du premier matériau. Le premier matériau est de préférence un matériau élastomère thermoplastique de type SEBS. Le deuxième matériau est de préférence un matériau élastomère thermoplastique de type SEBS plus souple que le premier matériau. Le surmoulage se fait de préférence par injection thermoplastique.

La bougie d'intubation peut ainsi être fabriquée en une ou deux étapes industrielles rapides, par injection thermoplastique.

Ce procédé permet de fabriquer des bougies d'intubation selon le premier aspect de l'invention en très grandes séries. Les systèmes d'automatisation et de robotisation sont en outre bien adaptés à ce type de procédé. Ce procédé permet d'obtenir une très bonne reproductibilité et une qualité constante pour de telles bougies d'intubation.

Un tel procédé permet avantageusement de limiter les coûts de fabrication de la bougie d'intubation.

La présente demande décrit aussi une bougie d'intubation comprenant un corps et une partie distale s'étendant depuis le corps. La partie distale comprend au moins trois segments disposés successivement le long de la partie distale, deux segments successifs étant séparés par une discontinuité, de sorte à conférer à la partie distale une souplesse croissante depuis une extrémité proximale de la partie distale solidaire du corps jusqu'à une extrémité distale libre de la partie distale, opposée à la première extrémité et destinée à pénétrer les voies aériennes supérieures d'un patient.

La discontinuité séparant deux segments consécutifs étant au moins l'une parmi une discontinuité de matière et une discontinuité de dimension, typiquement une discontinuité de section. En un point donné de la partie distale, la section est prise selon un plan perpendiculaire à la tangente en ce point. Par exemple, la discontinuité est formée par une réduction non continue de section et par un changement de matière. Selon un autre mode de réalisation, la discontinuité est formée uniquement par une réduction non continue de section, sans changement de matière entre les deux segments adjacents.

La partie distale et le corps sont de préférence également discontinus, en section et/ou en matière. Cette discontinuité entre le corps et la partie distale permet de former une première articulation. La partie distale est ainsi articulée vis-à-vis du corps. Cela permet en outre de distinguer facilement la partie distale du corps de la bougie.

La bougie d'intubation selon l'invention est peu chère, du fait de son procédé de fabrication, et donc largement accessible et utilisable par de nombreux praticiens. Elle n'est pas traumatisante pour les patients. Elle est maniable et peut s'utiliser avec des laryngoscopes et des vidéo laryngoscopes. Elle est pilotable et agile, ce qui permet un accès trachéal simple, sans risques et, par voie de conséquence, rapide.

La bougie d'intubation selon l'invention peut donc être utilisée dans des situations nécessitant un accès trachéal rapide, notamment en médecine d'urgence.

La bougie d'intubation selon l'invention peut également être utilisée chez des patients présentant des critères prédictifs d'intubation trachéale difficile.

### BREVE INTRODUCTION DES FIGURES

D'autres caractéristiques, buts et avantages de la présente invention apparaitront à la lecture de la description détaillée qui suit, et en regard des dessins annexés donnés à titre d'exemples non limitatifs et sur lesquels :
[Fig. 1] La figure 1 illustre une situation d'intubation ;
[Fig. 2] La figure 2 illustre la position d'une glotte difficile d'accès dans un champ de vision endoscopique d'un vidéolaryngoscope ;
[Fig. 3] La figure 3 montre une vue filaire de la bougie d'intubation selon un premier mode de réalisation de l'invention ;
[Fig. 4] La figure 4 montre une vue filaire de la bougie d'intubation selon un deuxième mode de réalisation de l'invention ;
[Fig. 5] La figure 5 illustre une bougie d'intubation selon un mode de réalisation de l'invention dans une situation d'intubation difficile assistée par un vidéolaryngoscope ;
[Fig. 6] La figure 6 illustre l'agilité d'une bougie d'intubation selon un mode de réalisation de l'invention.

### DESCRIPTION DETAILLEE

L'invention selon son premier aspect comprend notamment les caractéristiques optionnelles ci-après pouvant être utilisées en association ou alternativement :
Selon un mode de réalisation, les au moins trois segments présentent respectivement au moins trois souplesses segmentaires différentes entre elles, de sorte que la partie distale présente une souplesse croissante, éventuellement discontinue. Les souplesses segmentaires sont ainsi croissantes au fur et à mesure que l'on se rapproche de l'extrémité libre de la partie distale. La partie distale ne présente pas forcément une souplesse discontinue. Cela favorise une flexion rétrograde de la partie distale, depuis la deuxième extrémité libre jusqu'à la première extrémité, lors d'une progression de la bougie dans les VAS.

Selon un mode de réalisation, la souplesse croissante de la partie distale est induite par une diminution de section transversale desdits au moins trois segments les uns par rapport aux autres.

La diminution de section transversale peut se faire par paliers ou de façon progressive et sensiblement constante.

Selon l'invention, les au moins trois segments sont articulés les uns par rapport aux autres.

Selon un mode de réalisation, la bougie présente une discontinuité entre le corps et la partie distale. Cette discontinuité peut être formée par un changement de matériaux, un changement de dimension - typiquement un changement de section dans un plan perpendiculaire à l'axe terminal T. Cette discontinuité peut également être matérialisée par une réduction de section, tel qu'une réduction de diamètre, ou par une première nervure.

Selon un mode de réalisation, une articulation entre deux segments adjacents parmi les au moins trois segments est induite par un changement brusque de section transversale entre chacun desdits deux segments adjacents. Typiquement, ce changement brusque est formé par, de préférence uniquement par, une variation non continue ou par un chanfrein formant par exemple un angle avec chacun desdits deux segments adjacents compris entre 30° et 90°. La variation non continue entre un premier segment et un deuxième segment adjacents est par exemple telle que, pour un premier segment en regard de la première extrémité de la partie distale et présentant un diamètre Φ1, le deuxième segment en regard de la deuxième extrémité de la partie distale présente un diamètre Φ2 < 0,8.Φ1.

Selon un mode de réalisation préféré, une articulation entre deux segments adjacents parmi les au moins trois segments est formée par au moins une rainure. Cette rainure, dite rainure transverse peut par exemple s'étendre principalement dans un plan transversal à une direction principale d'extension de la partie distale au niveau de la rainure. Cette rainure transverse peut se présenter sous forme d'une gorge entre deux segments adjacents cylindriques. Cette gorge peut s'étendre sur tout le pourtour de la partie distale de sorte à définir un contour fermé. Alternativement cette gorge peut s'étendre sur une partie seulement du pourtour de la partie distale de sorte à définir un contour ouvert. La largeur de cette rainure est inférieure à la longueur de chacun des segments qu'elle sépare.

Selon un mode de réalisation, au moins un segment parmi les au moins trois segments est articulé et présente une souplesse segmentaire permettant un repli par retournement de la deuxième extrémité libre de la partie distale, par exemple en cas de blocage de ladite extrémité libre contre une structure anatomique.

Cela permet d'améliorer l'agilité de la partie distale et, de fait, de la bougie d'intubation. Cela permet également d'augmenter la sécurité d'utilisation de la bougie en améliorant le caractère anti-traumatique de la partie distale.

Selon un mode de réalisation, le corps présente un axe longitudinal terminal T et, au moins lorsque aucune force n'est appliquée sur la bougie d'intubation, au moins un segment parmi les au moins trois segments est désaxé par rapport à cet axe terminal T du corps de la bougie d'intubation.

Ce désaxage permet d'atteindre des glottes décentrées dans un champ de vision endoscopique d'un vidéolaryngoscope.

Selon un mode de réalisation, le désaxage de l'au moins un segment parmi les au moins trois segments élastomères par rapport à l'axe terminal T du corps de la bougie d'intubation forme un angle α compris entre 1° et 80° de préférence entre 10° et 30°. Cet angle est de préférence supérieur ou égal à 20°.

Selon un mode de réalisation, le corps de la bougie comprend une partie proximale s'étendant selon un axe longitudinal proximal B, et une partie terminale s'étendant selon un axe longitudinal terminal T et connectée à la partie distale de la bougie. L'axe terminal T forme un angle β par rapport à l'axe longitudinal proximal B compris entre 1° et 90°, de préférence entre 3° et 20°. Cet angle est de préférence supérieur ou égal à 5°. Le segment de la partie distale à proximité immédiate de la partie terminale du corps peut présenter un désaxage par rapport à l'axe terminal T selon un angle α compris entre 1° et 80°, de préférence entre 10° et 30°. Cet angle est de préférence supérieur ou égal à 20°.

Selon un mode de réalisation, la partie terminale du corps de la bougie comprend une succession de courbures, par exemple en forme de S.

Selon un mode de réalisation, la deuxième extrémité libre de la partie distale comprend un élément d'accroche tel qu'une boule terminale.

Une boule terminale présentant un diamètre supérieur au diamètre du dernier segment parmi les au moins trois segments, dit segment terminal, permet de former un élément d'accroche atraumatique.

De préférence, la deuxième extrémité libre de la partie distale est formée par l'élément d'accroche. Ainsi, la bougie d'intubation ne s'étend pas au-delà de l'élément d'accroche.

Selon un mode de réalisation, l'élément d'accroche est désaxé par rapport à un deuxième axe longitudinal S d'un segment terminal parmi les au moins trois segments.

Ce désaxage est de préférence orienté de façon à compenser approximativement une déviation générée par un premier désaxage entre le corps de la bougie et l'un au moins parmi les au moins trois segments de la partie distale. Cela permet de favoriser l'accroche de structures anatomiques. Cela permet également d'optimiser le franchissement des chicanes de la trachée.

L'élément d'accroche est solidaire d'une portion d'extrémité qui s'étend selon un axe différent du deuxième axe longitudinal S. Ainsi, le segment terminal présente au moins un coude. Une portion proximale du segment terminal s'étend depuis un segment adjacent au segment terminal et jusqu'au coude. Cette portion proximale du segment terminal s'étend selon le deuxième axe longitudinal S. Le segment terminal comprend également une portion distale. Cette portion distale s'étend entre le coude et l'élément d'accroche. Cette portion distale s'étend selon un axe différent de l'axe longitudinal S.

Selon un mode de réalisation, le désaxage de l'élément d'accroche par rapport au deuxième axe longitudinal (S) d'un segment terminal parmi les au moins trois segments forme un angle γ supérieur ou égal à 30° et de préférence compris entre 30° et 90°. Cet angle est de préférence supérieur ou égal à 60°.

Selon un mode de réalisation, le corps comprend au moins une cannelure longitudinale sur une partie de sa longueur.

Selon un mode de réalisation, la bougie comprend en outre un gaine de ventilation configurée pour coopérer avec l'au moins une cannelure longitudinale de façon à permettre un passage de fluide vers la partie distale.

L'au moins une cannelure facilite la ventilation du patient, notamment lorsqu'elle coopère avec une gaine de ventilation ajustée au corps de la bougie. Elle diminue ainsi le risque de suffocation du patient qui peut être oxygéné durant l'intervention du praticien. Elle facilite également une préhension du corps de la bougie pour le praticien, en créant un effet antidérapant. Elle améliore donc la pilotabilité de la bougie. Elle permet en outre de faciliter un glissement relatif entre la paroi d'une sonde d'intubation et le corps de la bougie introduite dans ladite sonde d'intubation, par diminution des frottements. Elle améliore donc la maniabilité de la bougie.

Selon l'invention, la partie distale comprend un noyau formé par les au moins trois segments et une gaine entourant le noyau et formée en un matériau différent de celui desdits au moins trois segments.

La gaine est surmoulée autour du noyau. Elle permet d'éviter un contact direct entre les au moins trois segments et les muqueuses ou les structures anatomiques des VAS.

Elle rend la partie distale moins traumatisante pour le patient. Elle permet en outre de modifier les propriétés mécaniques de la partie distale, en faisant par exemple office de ressort de rappel lorsque la partie distale est déformée.

Selon un mode de réalisation, la gaine surmoulée s'insère dans les rainures ou les gorges formant les articulations entre les segments adjacents de la partie distale. L'au moins une rainure transverse est remplie par le matériau formant la gaine et forme dès lors au moins un coussinet intersegmentaire. Ces coussinets intersegmentaires ont une fonction comparable à celle des disques intervertébraux d'une colonne vertébrale. Ils permettent de renforcer les qualités d'agilité, de maniabilité, de sécurité anti traumatique de la bougie. Ils procurent en outre une ténacité accrue aux articulations intersegmentaires. Ils augmentent également la raideur des articulations, ce qui induit une force de rappel supplémentaire lors de la déformation de la partie distale. La partie distale présente ainsi une bonne mémoire de forme et une bonne réactivité à la déformation.

Selon un mode de réalisation, la gaine se prolonge vers la deuxième extrémité libre de la partie distale de manière à former au moins un autre segment en un matériau différent de celui desdits au moins trois segments.

Ce prolongement permet d'amortir un contact avec une structure anatomique en particulier lors d'un blocage dans la progression de la bougie d'intubation.

Selon un mode de réalisation, le noyau se prolonge sensiblement jusqu'à la deuxième extrémité libre de la partie distale.

Les efforts appliqués par le praticien au corps sont transmis à la deuxième extrémité libre de la partie distale de façon optimisée. La pilotabilité de la bougie est améliorée.

Selon un mode de réalisation, la bougie d'intubation comprend en outre une poignée de pilotage au niveau de la partie proximale du corps.

Cela permet d'améliorer la pilotabilité de la bougie.

Selon un mode de réalisation, la poignée de pilotage est montée coulissante sur le corps de la bougie. De préférence elle est montée coulissante sur la partie proximale et sur une portion du corps.

Une poignée coulissante permet une utilisation de la bougie avec une prise en main initiale au plus proche de la partie distale. La poignée peut ensuite être déplacée vers la partie proximale du corps au fur et à mesure de la progression de la bougie dans les VAS. Cela garantit une maniabilité et une pilotabilité optimisées tout au long de la progression de la bougie dans les VAS.

Selon un mode de réalisation, la poignée de pilotage est montée de manière amovible sur le corps de la bougie.

Selon un mode de réalisation, les au moins trois segments sont en un matériau élastomère, de préférence en un matériau élastomère thermoplastique de type SEBS.

L'invention selon son deuxième aspect comprend notamment les caractéristiques optionnelles ci-après pouvant être utilisées en association ou alternativement :
Selon un mode de réalisation, le procédé de fabrication comprend en outre un surmoulage des au moins trois segments de la partie distale de sorte à former une partie distale comprenant un noyau en un premier matériau, de préférence obtenu par moulage, et une gaine en un deuxième matériau, de préférence obtenue par surmoulage, le deuxième matériau étant de préférence différent du premier matériau.

Selon un mode de réalisation, le premier matériau est un matériau élastomère, de préférence un matériau élastomère thermoplastique de type SEBS. Le deuxième matériau peut être un matériau élastomère du même type que le premier matériau. Il présente de préférence une dureté plus faible que celle du premier matériau. Il permet ainsi d'obtenir un effet de contact doux, dit « soft touch », totalement atraumatique au contact des parois anatomiques fragiles.

Dans la suite de la description et des revendications, les termes « guide », « mandrin » et « bougie » sont utilisés en synonymes. La bougie d'intubation est configurée pour guider la sonde d'intubation dans les VAS. La sonde d'intubation est creuse et tubulaire. Elle permet d'amener l'oxygène au patient intubé. A contrario, la bougie d'intubation n'est pas tubulaire, ni creuse. Elle est de préférence pleine ou nervurée longitudinalement. Elle est prévue pour coopérer avec la sonde d'intubation. L'homme du métier sait parfaitement distinguer une bougie d'intubation d'une sonde d'intubation. Ainsi, une sonde d'intubation est nécessairement creuse et tubulaire pour l'homme du métier, implicitement. De même, implicitement, une bougie d'intubation n'est pas creuse pour l'homme du métier.

Dans la présente invention, la souplesse de la partie distale peut être obtenue selon différents modes de réalisation éventuellement combinables entre eux.

La souplesse de la partie distale est croissante en direction de l'extrémité distale libre de la partie distale.

Cela signifie que pour une portion de longueur donnée la partie distale présente une souplesse croissance au fur et à mesure que l'on rapproche cette portion de l'extrémité distale.

Ainsi si une portion P1 de la partie distale présente une longueur L1 et une souplesse S1, une portion P2 de longueur L2=L1, située entre la portion P1 et l'extrémité distale de la partie distale présente une souplesse S2, telle que S2 > S1.

La longueur de la portion est mesurée selon la courbe selon laquelle la portion distale s'étend principalement.

Par exemple et de façon non limitative, les segments eux-mêmes peuvent présenter chacun une souplesse dite souplesse segmentaire. Cette souplesse segmentaire peut être induite par le matériau formant le segment. Ce matériau peut notamment présenter des propriétés mécaniques par exemple comparables à celles d'un élastomère et permettant une déformation élastique du segment. Pour un matériau donné, la forme et les dimensions du segment, en particulier sa longueur et son diamètre, peuvent influer sur la capacité à se déformer du segment et donc sur sa souplesse segmentaire.

La souplesse de la partie distale peut en outre être due au moins partiellement à une ou plusieurs articulations intersegmentaires.

La souplesse croissante de la partie distale en direction de son extrémité distale libre peut par exemple signifier que pour des segments adjacents Sn-1, Sn et Sn+1, le segment Sn étant disposé entre le segment Sn-1 et le segment Sn+1, et le segment Sn+1 étant disposé du côté de l'extrémité distale libre, c'est-à-dire entre le segment Sn et l'extrémité distale de la partie distale, la déformation de la partie distale au niveau du segment Sn est relativement plus importante qu'au niveau du segment Sn-1, et la déformation de la partie distale au niveau du segment Sn+1 est relativement plus importante qu'au niveau du segment Sn.

En particulier, en maintenant fixe le segment Sn et en appliquant une force au niveau d'une extrémité du segment Sn+1 d'un côté opposé au segment Sn (i.e., en appliquant une force identique sur l'extrémité distale du segment Sn+1), la flexion du segment Sn+1 sera plus importante que la flexion du segment Sn obtenue en maintenant fixe le segment Sn-1 et en appliquant la même force au niveau d'une extrémité du segment Sn d'un côté opposé au segment Sn-1 (i.e., en appliquant une force identique sur l'extrémité distale du segment Sn).

La souplesse de la partie distale peut également être vue comme étant fonction de ou correspondant à la capacité d'un segment Sn, en particulier à la capacité de l'extrémité de ce segment Sn, à se déplacer par rapport au segment Sn-1 adjacent. Ce déplacement est pris dans un plan perpendiculaire à l'axe longitudinal le long duquel les segments s'étendent, typiquement l'axe S. Ce déplacement ne se fait évidemment pas le long de ou parallèlement à l'axe longitudinal S. Les segments ne sont pas prévus pour coulisser les uns par rapport aux autres. La bougie est configuré pour qu'il n'y ait pas de translation d'un segment par rapport à un autre segment de la partie distale. La partie distale n'est pas télescopique.

On pourra par exemple mesurer la souplesse de la partie distale en mesurant des déplacements des segments relativement les uns par rapport aux autres, de la façon suivante :
Pour le segment Sn-1 situé entre le corps et le segment Sn, en maintenant le corps et en appliquant une force F à l'extrémité du segment Sn-1 opposée au corps, un premier déplacement de l'extrémité du segment Sn-1 est mesuré. Ce premier déplacement correspond à la flèche f1 du segment Sn-1 sollicité en flexion pour la force F.

Pour le segment Sn situé entre le segment Sn-1 et le segment Sn+1, en maintenant le segment Sn-1 et en appliquant la force F à l'extrémité du segment Sn opposée au segment Sn-1, un deuxième déplacement de l'extrémité du segment Sn est mesuré. Ce deuxième déplacement correspond à la flèche f2 du segment Sn sollicité en flexion pour la force F.

Pour le segment Sn+1 situé entre le segment Sn et l'extrémité libre de la partie distale, en maintenant le segment Sn et en appliquant la force F à l'extrémité du segment Sn+1 opposée au segment Sn, un troisième déplacement de l'extrémité du segment Sn+1 est mesuré. Ce troisième déplacement correspond à la flèche f3 du segment Sn+1 sollicité en flexion pour la force F.

Si f3 > f2 > f1, alors la souplesse de la partie distale est croissante en direction de son extrémité libre.

Ainsi dans la présente description on pourra remplacer le terme souplesse de la partie distale ou d'un segment par le terme déplacement, capacité de déplacement, déformation, capacité de déformation, flexion, capacité de flexion d'un segment.

Dans la suite, l'échelle de dureté Shore est une échelle utilisée pour mesurer la dureté des élastomères et de certaines matières plastiques.

On entend par « maniabilité » notamment la facilité de prise en main du guide d'intubation par l'opérateur en vue d'une introduction dans les VAS. La maniabilité peut s'apparenter à l'ergonomie générale du guide et résulte principalement de sa forme générale.

On entend par « pilotabilité » notamment les capacités du guide à diriger son extrémité distale dans les trois directions de l'espace à partir d'un effort exercé par l'opérateur sur une partie proximale du guide d'intubation.

On entend par « agilité » notamment la capacité de l'extrémité distale à passer des obstacles, des chicanes, à s'insinuer à l'intérieur des orifices et à se déformer pour épouser des ruptures d'axe de progression successifs relativement rapprochés. L'agilité peut également s'entendre de la capacité de l'extrémité distale du guide à atteindre des orifices désaxés d'une distance comprise entre 1 cm et 4 cm de l'axe longitudinal principal du corps du guide.

L'agilité peut également s'entendre de la capacité de l'extrémité distale à se retourner pour former sensiblement un angle de 180° avec son orientation initiale, et/ou à s'aplatir.

L'agilité peut donc représenter la capacité de l'extrémité distale à progresser sans blocage au travers de rétrécissements successifs dans une configuration initiale ou dans une configuration éversée.

Les termes « sensiblement », « environ », « de l'ordre de » signifient « à 10 % près » ou, lorsqu'il s'agit d'une orientation angulaire, « à 10° près ».

Lorsque deux éléments, par exemple des segments, sont désaxés, on entend qu'en position neutre, c'est-à-dire sans sollicitation appliquée sur la bougie d'intubation (autre que la gravité), ces deux éléments ne s'étendent pas selon le même axe.

Les différents désaxages et angles α, β, γ ne se font pas nécessairement dans un même plan. Ils sont de préférence choisis dans des plans différents.

Nous allons à présent décrire la présente invention au travers de modes de réalisation préférés mais non limitatifs.

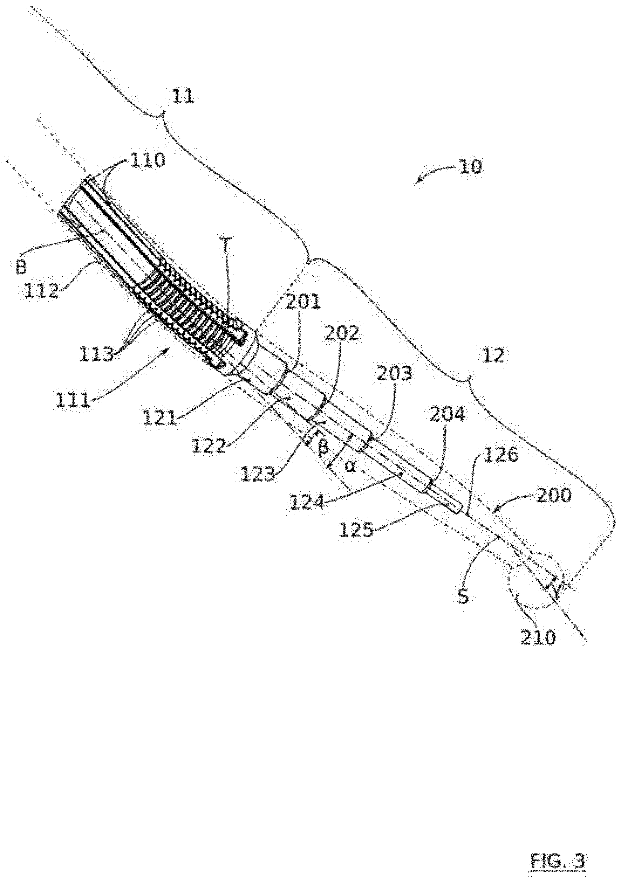

Selon un premier mode de réalisation de la bougie d'intubation illustré à la figure 3, la bougie 10 comprend un corps 11 présentant une longueur comprise entre 25 cm et 80 cm, et une partie distale 12 de quelques centimètres, destinée à être introduite dans les voies aériennes supérieures du patient.

Ce corps 11 se présente sous forme d'une tige s'étendant principalement selon un axe longitudinal B.

Le corps 11 comprend une partie proximale (non représentée) destinée à rester en dehors des voies aériennes supérieures du patient, et une partie terminale 111.

La partie terminale 111 s'étend au moins selon un axe longitudinal terminal T au niveau d'un côté destiné à être solidariser avec la partie distale 12.

La partie terminale 111 peut donc se présenter sous forme d'une partie intermédiaire entre la partie proximale du corps 11 et la partie distale 12 de la bougie.

Cette partie terminale 111 est de préférence désaxée par rapport à l'axe longitudinal B. Elle peut comprendre une ou plusieurs courbures, selon un même sens formant par exemple un C, ou selon des sens opposés formant par exemple un S.

L'axe terminal T forme par exemple un angle α d'environ 20° par rapport à l'axe longitudinal B.

Cette partie terminale 111 permet ainsi d'obtenir un premier degré de maniabilité de la bougie 10.

La partie terminale 111 du corps 11 est connectée à la partie distale 12.

A mesure que l'introduction de la bougie 10 progresse dans les VAS, la position de préhension du praticien se déplace sur le corps 11 vers la partie proximale.

La longueur du corps 11 peut avantageusement être définie et choisie en fonction de l'application, par exemple avec un laryngoscope de Macintosh, ou pour une intubation difficile avec une assistance à l'intubation sous videolaryngoscopie. Le diamètre externe de la bougie 10 est de préférence compris entre 3 mm et 4 mm. En particulier, une bougie présentant un diamètre de 3 mm est plus adaptée à une utilisation pédiatrique et une bougie présentant un diamètre de 4 mm est plus adaptée à une utilisation chez l'adulte.

Le corps 11 permet de transmettre à la partie distale 12 de la bougie les forces appliquées au niveau de la position de préhension.

Le maniement et/ou le pilotage de la bougie 10 se fait uniquement par l'intermédiaire du corps 11 dès lors que la partie distale 12 est introduite dans les VAS.

La progression de la bougie 10 dans les VAS peut se faire par un mouvement hélicoïdal.

Le corps 11 est donc configuré pour transmettre à la partie distale 12 les mouvements de translation et de rotation exercés au niveau de la position de préhension avec le moins d'inertie possible et le plus de précision possible.

Le corps 11 est de préférence en élastomère thermoplastique, par exemple en Styrène Etylène Butylène Styrène (SEBS).

Il présente de préférence une souplesse avec une mémoire de forme importante et une dureté comprise entre 60 et 80 shore D.

La bougie 10 peut avoir été préalablement introduite dans une sonde d'intubation avant intubation du patient, en particulier dans le cas d'une intubation effectuée avec un vidéo laryngoscope. Dans ce cas, la sonde d'intubation sous forme de tube, gaine le corps 11 de la bougie et au moins en partie la partie distale 12, ne laissant apparaitre éventuellement que la boule terminale 210.

Le corps 11 peut avantageusement comprendre des rainures 110 longitudinales.

Ces rainures 110 permettent de ménager un passage de fluide au sein du corps 11 de la bougie 10, en particulier de façon à permettre une ventilation du patient pendant l'intubation.

Dans ce cas, le corps 11 est de préférence recouvert sur toute sa longueur par une gaine de ventilation 112 thermorétractable et ajustée sur le pourtour du corps 11. Cette gaine de ventilation 112 forme ainsi avec les cannelures 110 un réseau de canalisations apte à véhiculer le fluide, par exemple de l'oxygène, au sein de la bougie 10. La partie terminale 111 du corps 11 comprend de préférence des cannelures transversales 113 permettant de maintenir la gaine de ventilation 112 thermorétractée.

Les rainures 110 permettent en outre de réduire les frottements entre le corps 11 et la sonde gainant le corps 11. La maniabilité et l'agilité de l'ensemble sont améliorées.

Les rainures 110 du corps 11 permettent aussi d'améliorer la préhension du praticien, en particulier pour effectuer des mouvements de rotation autour de l'axe longitudinal B.

De façon préférée mais optionnelle uniquement, la bougie 10 peut être équipée d'une poignée coulissante destinée à améliorer la préhension et le pilotage de la bougie 10.

Cette poignée coulissante est de préférence amovible.

Elle coulisse en translation le long du corps 11 selon l'axe longitudinal B.

Elle peut être bloquée dans une position de préhension par un système de serrage rapide.

Une telle poignée permet de piloter précisément et efficacement la bougie 10, à différentes positions de préhension tout au long de l'introduction de la bougie 10 dans les VAS.

Les rainures 110 du corps 11 peuvent avantageusement servir de guide ou de rail pour le coulissement en translation de la poignée coulissante, et permettre un blocage en rotation de la poignée coulissante assurant un renvoi immédiat et précis de l'effort appliqué par le praticien sur la poignée.

Cela permet par exemple de maintenir fermement un angle autour de l'axe longitudinal B tout en poussant la bougie plus avant dans les VAS.

La partie distale 12 de la bougie 10 peut comprendre une succession de segments 121, 122, 123, 124, 125 élastomères liés les uns aux autres depuis la première extrémité de la partie distale 12 solidaire de la partie terminale 111 du corps 11 vers la deuxième extrémité libre de la partie distale 12. La première extrémité de la partie distale 12 correspond ainsi à une extrémité proximale de la partie distale 12. La deuxième extrémité de la partie distale 12, également désignée extrémité libre, correspond ainsi à une extrémité distale de la partie distale 12.

La souplesse est croissante le long de cette succession de segments 121, 122, 123, 124, 125.

Selon un mode de réalisation préféré, chaque segment élastomère présente par exemple une souplesse supérieure au segment qui le précède immédiatement.

Pour des segments en matériau élastomère thermoplastique, l'augmentation de souplesse peut être obtenue par une variation dégressive des diamètres transverses, éventuellement combinée avec une augmentation des longueurs, desdits segments 121, 122, 123, 124, 125.

Pour un corps 11 de diamètre sensiblement égal à 4 mm (intubation chez l'adulte), la partie distale 12 peut comprendre cinq segments 121, 122, 123, 124, 125 élastomères thermoplastiques de préférence articulés les uns vis-à-vis des autres.

Pour un corps 11 de diamètre sensiblement égal à 3 mm (intubation pédiatrique), la partie distale 12 peut comprendre quatre segments121, 122, 123, 124 élastomères thermoplastiques de préférence articulés les uns vis-à-vis des autres.

La partie distale 12 de la bougie 10 comprend par exemple un premier segment 121 plus souple que le corps 11.

Ce premier segment 121 peut présenter une longueur comprise entre 3 et 4 mm et une forme cylindrique arrondie cintrée.

Il peut présenter un diamètre transverse sensiblement égal à 3,2 mm pour un corps 11 de diamètre sensiblement égal à 4 mm (intubation adulte), ou un diamètre transverse sensiblement égal à 2,7 mm pour un corps 11 de diamètre sensiblement égal à 3 mm (intubation pédiatrique).

Son axe longitudinal est de préférence incliné d'un angle compris entre 5° et 50° par rapport à l'axe terminal T de la partie terminale 111 du corps 11.

Il peut être courbé de façon à initier une déviation supplémentaire pour le segment 122 suivant.

Il peut être en un matériau élastomère thermoplastique tel que le SEBS.

Il peut présenter une souplesse plus importante que le corps 11 malgré une dureté shore identique comprise entre 60 et 80 shore D.

Ce premier segment 121 permet d'orienter la partie distale 12 selon une direction différente de celle portée par l'axe terminal T et/ou l'axe longitudinal B de la bougie 10.

Il permet en particulier d'accentuer ou de former un coude entre le corps 11 et la partie distale 12.

Cela favorise l'accès à des glottes 3 désaxées dans un champ de vision 21 endoscopique d'un vidéolaryngoscope.

Ce premier segment 121 confère à la partie distale 12 une agilité initiale.

La partie distale 12 de la bougie 10 comprend de préférence un deuxième segment 122 plus souple que le premier segment 121.

Ce deuxième segment 122 peut présenter une longueur comprise entre 3 et 5 mm et une forme cylindrique.

Il peut être dans l'axe et le prolongement du premier segment 121.

Il est de préférence en un matériau élastomère thermoplastique tel que le SEBS.

Il peut présenter un diamètre transverse sensiblement égal à 2,5 mm pour un corps 11 de diamètre sensiblement égal à 4 mm (intubation adulte), ou un diamètre transverse sensiblement égal à 2 mm pour un corps 11 de diamètre sensiblement égal à 3 mm (intubation pédiatrique).

Alternativement, il peut présenter un diamètre transverse moyen inférieur ou égal à 80% de celui du premier segment 121.

Il présente de préférence une souplesse plus importante que le premier segment 121, et une dureté shore identique comprise entre 60 et 80 shore D.

Le deuxième segment 122 est avantageusement articulé vis-à-vis du premier segment 121, par le biais d'une articulation 201.

Cette articulation 201 peut être obtenue par réduction brusque du diamètre transverse entre les premier et deuxième segments 121, 122. Cette réduction brusque est par exemple formée par un chanfrein dont la pente présente un angle compris entre 30° et 90°.

Cela permet de conférer une très bonne agilité et très bonne précision à la bougie.

La partie distale 12 de la bougie 10 comprend de préférence un troisième segment 123 plus souple que le deuxième segment 122.

Ce troisième segment 123 peut présenter une longueur comprise entre 4 et 6 mm et une forme cylindrique.

Il peut être dans l'axe et le prolongement du deuxième segment 122.

Il est de préférence en un matériau élastomère thermoplastique tel que le SEBS.

Il peut présenter un diamètre transverse sensiblement égal à 2,3 mm pour un corps 11 de diamètre sensiblement égal à 4 mm, ou un diamètre transverse sensiblement égal à 1,9 mm pour un corps 11 de diamètre sensiblement égal à 3 mm.

Alternativement, il peut présenter un diamètre transverse moyen inférieur ou égal à 80% de celui du deuxième segment 122.

Il présente de préférence une souplesse plus importante que le deuxième segment 122 et une dureté shore identique comprise entre 60 et 80 shore D.

Le troisième segment 123 est avantageusement articulé vis-à-vis du deuxième segment 122, par le biais d'une articulation 202.

Cette articulation 202 peut être obtenue par réduction brusque du diamètre transverse entre les deuxième et troisième segments 122, 123.

La partie distale 12 de la bougie 10 comprend de préférence un quatrième segment 124, de préférence plus souple que le troisième segment 123.

Ce quatrième segment 124 peut présenter une longueur comprise entre 4 et 6 mm et une forme cylindrique.

Il peut être dans l'axe et le prolongement du troisième segment 123.

Il est de préférence en un matériau élastomère thermoplastique tel que le SEBS.

Il peut présenter un diamètre transverse sensiblement égal à 1,8 mm pour un corps 11 de diamètre sensiblement égal à 4 mm, ou un diamètre transverse sensiblement égal à 1,2 mm pour un corps 11 de diamètre sensiblement égal à 3 mm.

Alternativement, il peut présenter un diamètre transverse moyen inférieur ou égal à 80% de celui du troisième segment 123.

Ce quatrième segment 124 peut être le segment terminal de la partie distale 12 de la bougie 10, en particulier d'une bougie 10 à usage pédiatrique dont le corps 11 présente un diamètre de 3 mm environ.

Le quatrième segment 124 présente de préférence une souplesse plus importante que le troisième segment 123 et une dureté shore identique comprise entre 60 et 80 shore D.

Il est avantageusement articulé vis-à-vis du troisième segment 123, par le biais d'une articulation 203.

Cette articulation 203 peut être obtenue par réduction brusque du diamètre transverse entre les troisième et quatrième segments 123, 124.

La partie distale 12 de la bougie 10 peut comprendre un cinquième segment 125, de préférence plus souple que le quatrième segment 124.

Ce cinquième segment 125 est dans cet exemple le segment terminal de la succession de segments 121, 122, 123, 124, 125.

Il peut présenter une longueur comprise entre 3 et 5 mm et une forme cylindrique.

Il peut être dans l'axe et le prolongement du quatrième segment 124, selon un axe longitudinal S.

Il est de préférence en un matériau élastomère thermoplastique tel que le SEBS.

Il peut présenter un diamètre transverse sensiblement égal à 1,5 mm du côté du quatrième segment 124, variant progressivement jusqu'à 1mm du côté opposé, pour un corps 11 de diamètre sensiblement égal à 4 mm.

Il présente de préférence une souplesse plus importante que le quatrième segment 124 et une dureté shore identique comprise entre 60 et 80 shore D.

Le cinquième segment 125 est avantageusement articulé vis-à-vis du quatrième segment 124, par le biais d'une articulation 204.

Cette articulation 204 peut être obtenue par réduction brusque du diamètre transverse entre les quatrième et cinquième segments 124, 125.

A titre indicatif, les principales dimensions des segments 121, 122, 123, 124, 125 et des articulations 201, 202, 203, 204 sont récapitulées dans le tableau ci-dessous :

| | Segments | | | | | Articulations | | | |
|---|---|---|---|---|---|---|---|---|---|
| N° | 121 | 122 | 123 | 124 | 125 | 201 | 202 | 203 | 204 |
| Diamètre (mm) | 3,24 | 2,50 | 1,75 | 1,00 | 0,50 | 2,50 à 3,24 | 1,75 à 2,50 | 1,00 à 1,75 | 0,50 à 1,00 |
| Longueur (mm) | 3,45 | 4,45 | 5,45 | 5,82 | 2,82 | 0,30 | 0,30 | 0,30 | 0,18 |

Selon ce premier mode de réalisation illustré à la figure 3, la partie distale 12 comprend une gaine 200 surmoulée autour des segments 121, 122, 123, 124, 125.

Cette gaine 200 surmoulée permet de lisser les changements brusques de sections transverses au niveau des articulations 201, 202, 203, 204 des différents segments 121, 122, 123, 124, 125.

Cela permet d'obtenir une déformation plus homogène de la partie distale 12. La souplesse est dès lors plus progressive le long de la partie distale 12. Cela permet également de rendre la partie distale 12 atraumatique.

La gaine 200 présente en particulier un diamètre sensiblement identique à celui du corps 11 au niveau du premier segment 121. Son diamètre diminue progressivement le long des segments 121, 122, 123, 124, 125 pour atteindre 2,8 mm environ au niveau du segment terminal.

Elle peut être réalisée en un matériau différent du corps 11 et des segments 121, 122, 123, 124, 125.

Elle peut être plus souple et présenter une dureté comprise entre 60 et 80 shore A par exemple.

Elle est de préférence réalisée en un matériau élastomère thermoplastique avec une finition de type « soft touch », i.e. un état de surface lisse et doux.

La gaine 200 se prolonge de préférence le long de l'axe S au-delà du segment terminal.

Elle peut former un autre segment 126 de diamètre sensiblement égal à 2,8 mm comprenant une boule terminale 210 de diamètre compris entre 4 mm et 5 mm.

Cette boule terminale 210 correspond à l'extrémité libre de la partie distale 12.

La boule terminale 210 permet d'accrocher une structure anatomique telle qu'une corde vocale 6.

Elle est de préférence désaxée d'un angle γ par rapport à l'axe longitudinal S.

Ce désaxage peut être orienté de façon à compenser la déviation générée par le premier segment 121.

De préférence, ce désaxage forme un angle γ supérieur ou égal à 45° de préférence supérieur ou égale à 50°. Selon un exemple, cet angle est compris entre 50° et 55°.

Cela permet d'améliorer considérablement la maniabilité et la précision de la bougie d'intubation.

La boule terminale 210 est de préférence orientée d'un côté opposé au côté intérieur du coude formé par la partie distale 12 et le corps 11.

Cela permet d'optimiser l'accroche de structures anatomiques et d'améliorer le franchissement de chicanes.

Le matériau élastomère de gaine 200 est de préférence translucide, et de préférence de couleur bleue.

Cela permet de transmettre et de diffuser une lumière émise par une diode électroluminescente du laryngoscope ou du vidéolaryngoscope par exemple.

La lumière ainsi diffusée par la gaine 200 de la partie distale 12 permet d'éclairer les parois et/ou les structures anatomiques avoisinantes.

La lumière bleutée permet d'obtenir le meilleur contraste avec les structures anatomiques des VAS.

Il en résulte pour le praticien utilisant un laryngoscope classique, une visualisation directe de la partie distale 12 et notamment de sa boule terminale 210.

Pour le praticien utilisant un vidéolaryngoscope, l'image de la partie distale 12 et/ou des structures anatomiques avoisinantes est plus lumineuse. Un effet d'irisation des parois anatomiques peut également être obtenu.

Cette visualisation de la partie distale 12 au fur et à mesure de sa progression entre les structures anatomiques des VAS, procure un avantage majeur sur la pilotabilité de la bougie 10.

Le geste médical du praticien est ainsi plus précis et donc plus rapide.

La combinaison des segments 121, 122, 123, 124, 125 et de la gaine 200 confère à la partie distale 12 une grande agilité.

En particulier, la partie distale 12 peut ainsi se plier progressivement, voire se retourner à 180° du côté de l'extrémité libre, lors de sa progression dans les VAS.

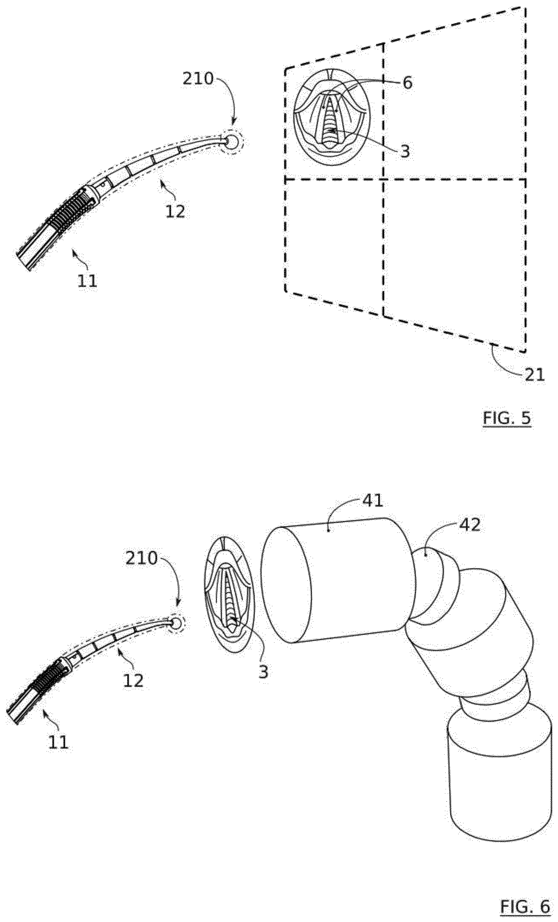

Cela permet notamment d'atteindre des glottes 3 désaxées comme illustré à la figure 5. La succession des segments 121, 122, 123, 124, 125 permet de former une partie distale 12 présentant une longueur totale de l'ordre de 4 cm.

Une telle partie distale 12 permet donc d'atteindre des glottes 3 désaxées d'une distance de désaxage inférieure ou égale à 4 cm par rapport à l'axe longitudinal B de la bougie 10, tandis qu'une bougie standard est limitée à une distance de désaxage inférieure à 2,8 cm.

Par ailleurs, les cartilages saillants de la région sous glottique (thyroïde 41 et cricoïde 42) peuvent bloquer la progression de la partie distale 12 comme illustré à la figure 6.

Le retournement de l'extrémité libre de la partie distale 12 permet alors d'éviter des lésions du larynx et/ou des autres parties anatomiques telles que les cordes vocales et les parois très fragiles de la trachée.

Le retournement forme dès lors une nouvelle extrémité contondante non traumatique.

Cette nouvelle extrémité reste souple et agile et permet de continuer la progression de la bougie 10 dans des orifices infra-centimétriques. Le diamètre minimum de franchissement d'une telle bougie 10 est avantageusement inférieur à 8 mm, tandis que celui-ci est supérieur à 12 mm pour une bougie standard.

La partie distale 12 d'une telle bougie permet ainsi d'améliorer à la fois la sécurité et l'efficacité de l'intubation. Le praticien peut dès lors intuber le patient de façon plus sereine, en minimisant voire en éliminant le risque de blessure. Le geste du praticien peut être plus assuré, plus volontaire, plus audacieux et par suite plus efficace et plus rapide.

D'autres modes de réalisation de la bougie d'intubation selon l'invention peuvent être envisagés. Seules les caractéristiques distinctes du premier mode de réalisation sont décrites ci-après, les autres caractéristiques non décrites étant réputées identiques à celles du premier mode de réalisation.

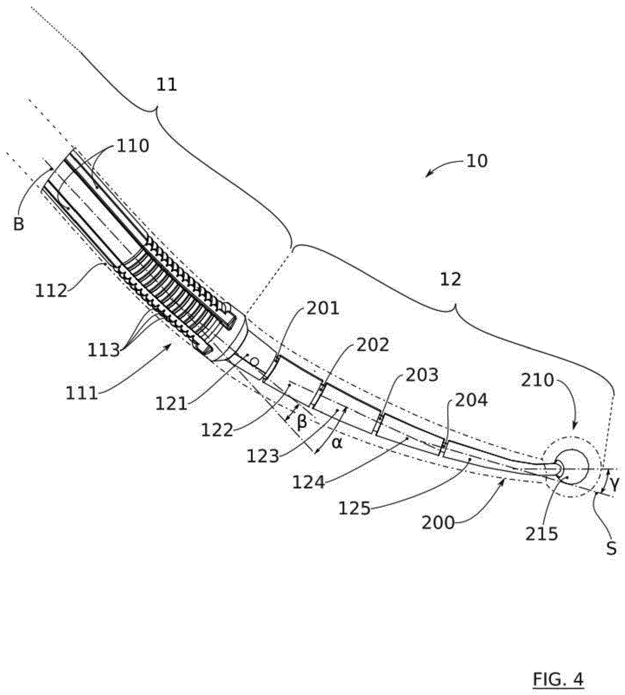

Selon un deuxième mode de réalisation de la bougie d'intubation illustré à la figure 4, la partie distale 12 de la bougie 10 peut comprendre cinq segments 121, 122, 123, 124, 125 tronconiques de préférence articulés les uns vis-à-vis des autres.

Le diamètre transverse de ces segments diminue donc progressivement depuis un côté du premier segment 121 lié à la partie terminale 111 du corps 11 jusqu'à une extrémité du segment terminal 125.

Pour un corps 11 de diamètre sensiblement égal à 4 mm (intubation chez l'adulte), le premier segment 121 peut ainsi présenter un diamètre transverse sensiblement égal à 3,2 mm au niveau de la jonction avec la partie terminale 111 du corps 11. Ce diamètre diminue progressivement le long des cinq segments 121, 122, 123, 124, 125 jusqu'à atteindre un diamètre transverse environ égal à 0,8 mm à l'extrémité du cinquième segment 125.

Cette diminution progressive et régulière du diamètre transverse le long des segments 121, 122, 123, 124, 125 de la partie distale 12 permet d'obtenir un comportement mécanique progressif au niveau de la partie distale 12.

L'agilité, la maniabilité et la pilotabilité de la bougie 10 s'en trouvent améliorées.

Selon une possibilité, le segment terminal 125 peut comprendre une boule terminale de cœur 215 à son extrémité, dans le même matériau que le segment 125.

Cette boule terminale de cœur 215 est de préférence désaxée par rapport à l'axe S du segment terminal. Typiquement, ce désaxage forme un angle γ supérieur ou égal à 45°, et de préférence supérieur ou égale à 50° par rapport à l'axe S. Selon un mode de réalisation, cet angle est compris entre 50° et 55°.

Cela permet notamment, après formation par surmoulage de la gaine 200 et de la boule terminale 210 en un deuxième matériau élastomère, d'obtenir une partie distale 12 comprenant un noyau et une gaine 200 sur environ toute la longueur de la partie distale 12.

Le comportement mécanique de cette partie distale 12 est ainsi amélioré. Il est en particulier plus homogène.

Les articulations 201, 202, 203, 204 entre les différents segments 121, 122, 123, 124, 125 peuvent être de simples rainures transverses, sur tout ou partie de la circonférence entre deux segments adjacents.

Ces rainures peuvent présenter une profondeur de quelques centaines de microns par exemple.

Typiquement, le ratio entre d'une part le diamètre des segments adjacents à la rainure, au niveau de leurs extrémités jouxtant cette rainure, et d'autre part le fond de la rainure est compris entre 1,2 et 3. Cela permet de conférer une très bonne agilité et très bonne précision à la bougie.

Une rainure partielle, et/ou une rainure sur toute la circonférence, et/ou plusieurs rainures peuvent former une articulation 201, 202, 203, 204.

Le noyau formé par les segments 121, 122, 123, 124, 125 articulés les uns par rapport aux autres par de telles articulations 201, 202, 203, 204 peut s'apparenter à une colonne vertébrale.

Le comportement mécanique de la partie distale 12 est ainsi amélioré.

Par ailleurs, lors de l'opération de surmoulage, les rainures 201, 202, 203, 204 situées entre les segments 121, 122, 123, 124, 125 de diamètres dégressifs sont remplies par le deuxième matériau élastomère formant la gaine 200.

Ce matériau élastomère ainsi intercalé dans les rainures 201, 202, 203, 204 forme des coussinets entre les segments 121, 122, 123, 124, 125. Ces coussinets présentent un comportement mécanique qui s'apparente à celui des disques intervertébraux d'une colonne vertébrale.

Ces coussinets intersegmentaires permettent d'optimiser à la fois la souplesse et le rappel par mémoire de forme de la partie distale 12.

La déformation de la partie distale 12 est reproductible pour chaque type d'appui de la partie distale 12 contre une paroi.

Pour le praticien la déformation de cette partie distale 12 devient donc prévisible lors d'un appui sur les parois anatomiques. Le praticien peut donc facilement évaluer la situation de la partie distale 12 lors de la progression à l'aveugle dans les VAS du patient.

En cas d'intubation difficile, le praticien peut alors manier efficacement la bougie 10 en fonction des sensations perçues d'après le comportement de la partie distale 12, sans risque pour le patient, de façon à trouver rapidement un cheminement jusqu'aux bronches au travers des VAS du patient.

Ce second mode de réalisation procure une agilité, une maniabilité et une pilotabilité accrue de la bougie 10.

Une telle bougie 10 permet au praticien de réaliser un geste médical moins hasardeux, plus ciblé car prédictible et donc plus rapide, lors de la progression dans les structures anatomiques pour intuber un patient.

L'invention selon son deuxième aspect concerne un procédé de fabrication d'une bougie 10. Ce procédé comprend de préférence les étapes suivantes :
Mouler par injection le corps 11 et les segments 121, 122, 123, 124, 125 de la partie distale 12 en un premier matériau élastomère thermoplastique, de préférence en SEBS médical. - Placer au moins les segments 121, 122, 123, 124, 125 précédemment moulés de la partie distale 12 dans un moule de surmoulage de la partie distale 12,

Surmouler par injection une gaine 200 en un deuxième matériau élastomère thermoplastique sur le noyau semi-rigide formé par les segments 121, 122, 123, 124, 125 de la partie distale 12. Ce deuxième matériau peut être un SEBS médical sensiblement identique au premier matériau. Selon une possibilité, il peut être différent de façon à privilégier la biocompatibilité et le contact atraumatique avec les muqueuses fragiles des VAS.

De façon alternative, mouler et surmouler par bi-injection le corps 11, et les segments 121, 122, 123, 124, 125, puis la gaine 200. Cela permet d'éviter le transfert et/ou le déplacement du corps 11 et des segments 121, 122, 123, 124, 125 vers un moule de surmoulage pour surmouler la gaine 200.

Au vu de la description qui précède, il apparaît clairement que l'invention offre une solution particulièrement efficace pour permettre un accès trachéal rapide et atraumatique, tout en présentant un coût limité. Elle s'avère particulièrement avantageuse dans les situations d'intubation difficiles.

L'invention n'est pas limitée aux modes de réalisation précédemment décrits mais s'étend à tous modes de réalisation entrant dans la portée des revendications.

Par exemple, la partie distale 12 peut comprendre des segments consécutifs présentant un brusque changement de section transverse et d'autres segments consécutifs présentant un diamètre transverse régulièrement et progressivement décroissant.

Différents types d'articulations 201, 202, 203, 204 peuvent être combinés au sein de la partie distale 12.

Le surmoulage de la gaine 200 peut se faire au niveau des segments 121, 122, 123, 124, 125 ou peut s'étendre au-delà du segment terminal 125.

## Revendications

1. Bougie d'intubation (10) présentant une tige comprenant un corps (11) et une partie distale (12) s'étendant depuis le corps (11), ladite partie distale (12) comprend au moins trois segments (121, 122, 123) distincts entres eux, disposés successivement et configurés de sorte à conférer à la partie distale une souplesse croissante depuis une première extrémité de la partie distale (12) solidaire du corps (1 1) jusqu'à une deuxième extrémité libre de la partie distale (12), opposée à la première extrémité et destinée à pénétrer les voies aériennes supérieures d'un patient, **caractérisée en ce que**:
- la souplesse croissante de la partie distale est induite par une diminution de section transversale desdits au moins trois segments (121, 122, 123) les uns par rapport aux autres,
- les au moins trois segments (121, 122, 123) sont articulés les uns par rapport aux autres, par au moins une articulation (201, 202, 203) conformée entre deux segments adjacents parmi les au moins trois segments (121, 122, 123), et
- les au moins trois segments (121, 122, 123) forment un noyau surmoulé par une gaine (200) lequel est formée en un matériau différent de celui desdits au moins trois segments (121, 122, 123).

2. Bougie d'intubation (10) selon la revendication précédente dans laquelle les au moins trois segments présentent respectivement au moins trois souplesses segmentaires différentes entre elles, de sorte que la partie distale présente une souplesse croissante discontinue.

3. Bougie d'intubation (10) selon la revendication précédente dans laquelle l'au moins une articulation (201, 202, 203) entre deux segments (121, 122) adjacents est formée par une variation non continue de section transversale entre chacun desdits deux segments adjacents.

4. Bougie d'intubation (10) selon la revendication 1 dans laquelle l'au moins une articulation (201, 202, 203) entre deux segments (122, 123) adjacents parmi les au moins trois segments (121, 122, 123) est formée par au moins une rainure transverse.

5. Bougie d'intubation (10) selon l'une quelconque des trois revendications précédentes dans laquelle au moins un segment parmi les au moins trois segments (121, 122, 123) est articulé et présente une souplesse segmentaire permettant un repli par retournement de la deuxième extrémité libre de la partie distale (12).

6. Bougie d'intubation (10) selon l'une quelconque des revendications précédentes dans laquelle le corps (1 1) présente un axe longitudinal, dit axe terminal (T), et dans laquelle au moins un segment parmi les au moins trois segments (121, 122, 123) est désaxé par rapport à l'axe longitudinal terminal (T) du corps (11) de la bougie d'intubation (10).

7. Bougie d'intubation (10) selon la revendication précédente dans laquelle le désaxage de l'au moins un segment parmi les au moins trois segments (121, 122, 123) par rapport à l'axe longitudinal terminal (T) du corps (11) de la bougie d'intubation (10) forme un angle α compris entre 1° et 80°, de préférence entre 10° et 30°.

8. Bougie d'intubation (10) selon l'une quelconque des revendications précédentes dans laquelle le corps (11) de la bougie (10) comprend une partie proximale s'étendant selon un axe longitudinal proximal (B), et une partie terminale (111) s'étendant selon un axe longitudinal terminal (T), ladite partie terminale (111) étant connectée à la partie distale (12) de la bougie (10), et dans laquelle l'axe longitudinal terminal (T) de la partie terminale (111) forme un angle β par rapport à l'axe longitudinal proximal (B) compris entre 1° et 90°, de préférence entre 3° et 20º.

9. Bougie d'intubation (10) selon l'une quelconque des revendications précédentes dans laquelle la deuxième extrémité libre de la partie distale (12) comprend un élément d'accroche (210) tel qu'une boule terminale.

10. Bougie d'intubation (10) selon la revendication précédente dans laquelle l'élément d'accroche (210) est désaxé par rapport à un axe longitudinal (S) d'un segment terminal parmi les au moins trois segments (121, 122, 123).

11. Bougie d'intubation (10) selon la revendication précédente dans laquelle le désaxage de l'élément d'accroche (210) par rapport à l'axe longitudinal (S) d'un segment terminal parmi les au moins trois segments (121, 122, 123) forme un angle γ supérieur ou égal à 30° et de préférence supérieur ou égal à 60°.

12. Bougie d'intubation (10) selon l'une quelconque des revendications précédentes dans laquelle le corps (1 1) comprend au moins une cannelure (110) longitudinale sur une partie de sa longueur.

## Patentansprüche

1. Intubationshilfe (10), die einen Schaft aufweist, der einen Körper (11) und einen sich vom Körper (11) erstreckenden distalen Teil (12) umfasst, wobei der distale Teil (12) mindestens drei Segmente (121, 122, 123) umfasst, die sich voneinander unterscheiden, nacheinander angeordnet sind und dazu konfiguriert sind, dem distalen Teil eine zunehmende Flexibilität von einem ersten Ende des distalen Teils (12), das fest mit dem Körper (11) verbunden ist, bis zu einem zweiten freien Ende des distalen Teils (12) zu verleihen, das dem ersten Ende gegenüberliegt und dazu bestimmt ist, in die oberen Atemwege eines Patienten einzudringen, **dadurch gekennzeichnet, dass**:
- die zunehmende Flexibilität des distalen Teils durch eine Verringerung des Querschnitts der mindestens drei Segmente (121, 122, 123) im Verhältnis zueinander bestimmt wird,
- die mindestens drei Segmente (121, 122, 123) durch mindestens ein Gelenk (201, 202, 203) miteinander gelenkig verbunden sind, das zwischen zwei benachbarten Segmenten unter den mindestens drei Segmenten (121, 122, 123) ausgebildet ist, und
- die mindestens drei Segmente (121, 122, 123) einen Kern bilden, der von einer Hülle (200) umspritzt ist, die aus einem anderen Material als die mindestens drei Segmente (121, 122, 123) hergestellt ist.

2. Intubationshilfe (10) nach dem vorhergehenden Anspruch, wobei die mindestens drei Segmente jeweils mindestens drei voneinander unterschiedliche Grade an segmentaler Flexibilität aufweisen, sodass der distale Teil eine diskontinuierlich zunehmende Flexibilität aufweist.

3. Intubationshilfe (10) nach dem vorhergehenden Anspruch, wobei das mindestens eine Gelenk (201, 202, 203) zwischen zwei benachbarten Segmenten (121, 122) durch eine nichtkontinuierliche Querschnittsänderung zwischen jedem von den beiden benachbarten Segmenten gebildet wird.

4. Intubationshilfe (10) nach Anspruch 1, wobei das mindestens eine Gelenk (201, 202, 203) zwischen zwei benachbarten Segmenten (122, 123) unter den mindestens drei Segmenten (121, 122, 123) durch mindestens eine Quernut gebildet wird.

5. Intubationshilfe (10) nach einem der drei vorhergehenden Ansprüche, wobei mindestens ein Segment unter den mindestens drei Segmenten (121, 122, 123) gelenkig ist und eine segmentale Flexibilität aufweist, die ein Zusammenklappen durch Drehen des zweiten freien Endes des distalen Teils (12) ermöglicht.

6. Intubationshilfe (10) nach einem der vorhergehenden Ansprüche, wobei der Körper (11) eine Längsachse aufweist, die als Endachse (T) bezeichnet wird, und wobei mindestens ein Segment unter den mindestens drei Segmenten (121, 122, 123) gegenüber der Endlängsachse (T) des Körpers (11) der Intubationshilfe (10) versetzt ist.

7. Intubationshilfe (10) nach dem vorhergehenden Anspruch, wobei der Versatz des mindestens einen Segments unter den mindestens drei Segmenten (121, 122, 123) in Bezug auf die Endlängsachse (T) des Körpers (11) der Intubationshilfe (10) einen Winkel α bildet, der zwischen 1° und 80°, vorzugsweise zwischen 10° und 30°, liegt.

8. Intubationshilfe (10) nach einem der vorhergehenden Ansprüche, wobei der Körper (11) der Einführhilfe (10) einen proximalen Teil, der sich entlang einer proximalen Längsachse (B) erstreckt, und einen Endteil (111) umfasst, der sich entlang einer Endlängsachse (T) erstreckt, wobei der Endteil (111) mit dem distalen Teil (12) der Einführhilfe (10) verbunden ist, und wobei die Endlängsachse (T) des Endteils (111) einen Winkel β zur proximalen Längsachse (B) bildet, der zwischen 1° und 90°, vorzugsweise zwischen 3° und 20°, liegt.

9. Intubationshilfe (10) nach einem der vorhergehenden Ansprüche, wobei das zweite freie Ende des distalen Teils (12) ein Einhakelement (210), wie beispielsweise eine Endkugel, umfasst.

10. Intubationshilfe (10) nach dem vorhergehenden Anspruch, wobei das Einhakelement (210) gegenüber einer Längsachse (S) eines Segments unter den mindestens drei Segmenten (121, 122, 123) versetzt ist.

11. Intubationshilfe (10) nach dem vorhergehenden Anspruch, wobei der Versatz des Einhakelements (210) gegenüber der Längsachse (S) eines Endsegments unter den mindestens drei Segmenten (121, 122, 123) einen Winkel γ bildet, der größer oder gleich 30° und vorzugsweise größer oder gleich 60° ist.

12. Intubationshilfe (10) nach einem der vorhergehenden Ansprüche, wobei der Körper (11) über einen Teil seiner Länge mindestens eine Längsnut (110) umfasst.

## Claims

1. An intubation bougie (10) having a shaft comprising a body (11) and a distal part (12) extending from the body (11), said distal part (12) comprising at least three segments (121, 122, 123) different from one another, arranged successively and configured to confer to the distal part increasing flexibility from a first end of the distal part (12), integral with the body (11), to a free second end of the distal part (12), opposite the first end and intended to penetrate the upper airways of a patient, **characterised in that**:
- the increasing flexibility of the distal part is determined by a decrease of the cross-section of said at least three segments (121, 122, 123) with respect to one another;
- the at least three segments (121, 122, 123) are articulated to one another by means of at least one articulation (201, 202, 203) formed between two adjacent segments from among the at least three segments (121, 122, 123), and
- the at least three segments (121, 122, 123) form a core overmoulded by a sheath (200), which is manufactured with a material different from that of said at least three segments (121, 122, 123).

2. The intubation bougie (10) according to the preceding claim, wherein the at least three segments have, respectively, at least three degrees of segmental flexibility different from one another, such that the distal part has discontinuous increasing flexibility.

3. The intubation bougie (10) according to the preceding claim, wherein the at least one articulation (201, 202, 203) between two adjacent segments (121, 122) is formed by a non-continuous variation in cross-section between each of said two adjacent segments.

4. The intubation bougie (10) according to claim 1, wherein the at least one articulation (201, 202, 203) between two adjacent segments (122, 123) from among the at least three segments (121, 122, 123) is formed by at least one transverse groove.

5. The intubation bougie (10) according to any of the three preceding claims, wherein at least one segment from among the at least three segments (121, 122, 123) is articulated and has a segmental flexibility that allows a folding by means of the rotation of the free second end of the distal part (12).

6. The intubation bougie (10) according to any of the preceding claims, wherein the body (11) has a longitudinal axis, referred to as terminal axis (T), and wherein at least one segment from among the at least three segments (121, 122, 123) is misaligned with respect to the terminal longitudinal axis (T) of the body (11) of the intubation bougie (10).

7. The intubation bougie (10) according to the preceding claim, wherein the misalignment of the at least one segment from among the at least three segments (121, 122, 123) with respect to the terminal longitudinal axis (T) of the body (11) of the intubation bougie (10) forms an angle α comprised between 1° and 80°, preferably between 10° and 30°.

8. The intubation bougie (10) according to any of the preceding claims, wherein the body (11) of the bougie (10) comprises a proximal part extending according to a proximal longitudinal axis (B), and a terminal part (111) extending according to a terminal longitudinal axis (T), with said terminal part (111) being connected to the distal part (12) of the bougie (10), and wherein the terminal longitudinal axis (T) of the terminal part (111) forms an angle β with respect to the proximal longitudinal axis (B) comprised between 1° and 90°, preferably between 3° and 20°.

9. The intubation bougie (10) according to any of the preceding claims, wherein the free second end of the distal part (12) comprises an engagement element (210), such as a terminal ball.

10. The intubation bougie (10) according to the preceding claim, wherein the engagement element (210) is misaligned with respect to a longitudinal axis (S) of one segment from among the at least three segments (121, 122, 123).

11. The intubation bougie (10) according to the preceding claim, wherein the misalignment of the engagement element (210) with respect to the longitudinal axis (S) of one terminal segment from among the at least three segments (121, 122, 123) forms an angle γ greater than or equal to 30° and, preferably, greater than or equal to 60°.

12. The intubation bougie (10) according to any of the preceding claims, wherein the body (11) comprises at least one longitudinal groove (110) on part of its length.
